# NEUE EUROPÄISCHE PATENTSCHRIFT

(11) **EP 0 543 874 B2**
(45) Veröffentlichungstag und Bekanntmachung des Hinweises auf die Entscheidung über den Einspruch: **08.09.1999**
(45) Hinweis auf die Patenterteilung: 06.12.1995
(21) Anmeldenummer: 91914552.4
(22) Anmeldetag: 10.08.1991
(51) Int. Cl.: C07C 209/84, C11D 1/62

(54) **RESTGEHALTVERMINDERUNG VON FREIEM ALKYLIERUNGSMITTEL IN WÄSSRIGEN LÖSUNGEN**
REDUCTION OF THE RESIDUAL CONTENT OF FREE ALKYLATION AGENTS IN AQUEOUS SOLUTIONS
REDUCTION DE LA TENEUR RESIDUELLE EN AGENTS LIBRES D'ALKYLATION DANS DES SOLUTIONS AQUEUSES

(30) Priorität: 18.08.1990 DE 4026184
(43) Veröffentlichungstag der Anmeldung: 02.06.1993
(73) Patentinhaber: Henkel Kommanditgesellschaft auf Aktien, 40191 Düsseldorf (DE)
(72) Erfinder: BEHLER, Ansgar, D-4250 Bottrop (DE); PLOOG, Uwe, D-5667 Haan (DE); HENSEN, Hermann, D-5667 Haan (DE); UPHUES, Günter, D-4019 Monheim (DE)
(86) Internationale Anmeldenummer: EP9101526
(87) Internationale Veröffentlichungsnummer: WO9203406

(56) Entgegenhaltungen:
- DD-A- 259 847
- DD-A- 279 475
- US-A- 4 845 289
- PATENT ABSTRACTS OF JAPAN, unexamined applications, C Field, Band 10, Nr. 28, 04. Februar 1986, The Patent Office Japanese Government, Seite 7 C 326, JP-A- 60 178 846
- SOVIET INVENTIONS ILLUSTRATED, Sektion Ch, Woche C 30, 03. September 1980, DERWENT PUBLICATIONS LTD., London, D 25; & SU-A-702 003
- AM. IND. HYG. ASSOC. J. 46(3), 1985, pages 111-114
- ORGANIC SYNTHESES, Collective Vol. 4, JOHN WILEY & SONS, INC. 1963, pages 674-676
- MARCH, J., "Advanced Organic Chemistry - 3rd Edition" 1985, JOHN WILEY & SONS

## Beschreibung

Die Erfindung betrifft ein Verfahren zur Verminderung des Restgehaltes an freiem Alkylierungsmittel in wäßrigen Lösungen kationischer Tenside durch Nachbehandlung der Lösungen mit ausgewählten Aminosäuren.

### Stand der Technik

Kationische Tenside vom Typ der quartären Ammoniumverbindungen stellen wichtige Produkte zur Herstellung von Wäscheweichspülem, Avivagemitteln für Haarbehandlungszwecke, Antistatika und Desinfektionsmitteln dar, Zu ihrer Synthese geht man in der Regel von tertiären Aminen aus, die mit einem Alkylierungsmittel, beispielsweise Methylchlorid, Benzylchlorid oder Dimethylsulfat umgesetzt werden, Um die Reaktion quantitativ durchzuführen, werden die Alkylierungsmittel dabei üblicherweise im Überschuß eingesetzt und anschließend nicht umgesetzte Mengen durch Zugabe von Ammoniak zerstört [**J.Falbe (ed.), "Surfactants in consumer products", Springer Verlag, Berlin, 1987, S.107-114**].

Da die Alkylierung nicht quantitativ erfolgt, weisen quartäre Ammoniumverbindungen, die auf den genannten Wegen hergestellt werden, ohne Nachbehandlung dennoch einen Restgehalt von 0,1 bis 3 Gew.-% des Alkylierungsmittels auf. Da es zur Erzielung toxikologisch unbedenklicher Produkte erforderlich ist, den Gehalt an Methylchlorid, Benzylchlorid oder Dimethylsulfat in den Produkten auf ein möglichst niedriges Niveau herabzusenken, hat es in der Vergangenheit nicht an Versuchen gemangelt, dieses Ziel durch eine geeignete Aufarbeitung sicherzustellen. Üblicherweise wird dabei der Gehalt an freiem Alkylierungsmittel durch mehrstündige thermische Behandlung bis auf einen geringen Betrag herabgesetzt. Diese Vorgehensweise ist jedoch mit einem hohen Aufwand an Zeit und Energie verbunden. Die Aufgabe der Erfindung bestand somit darin, ein neues Verfahren zur Nachbehandlung von kationischen Tensiden zu entwickeln, das eine deutliche Verminderung des Gehaltes an freiem Alkylierungsmittel sicherstellt und frei von den geschilderten Nachteilen ist.

### Beschreibung der Erfindung

Gegenstand der Erfindung ist ein Verfahren zur Verminderung des Restgehaltes an freiem Alkylierungsmittel in wäßrigen Lösungen kationischer Tenside, welches sich dadurch auszeichnet, daß man die Lösungen mit einer Aminosäuren mit 2 bis 8 Kohlenstoffatomen bei erhöhter Temperatur und gegebenenfalls erhöhtem Druck nachbehandelt.

Überraschenderweise wurde gefunden, daß diese Art der Aufarbeitung gegenüber der thermischen Nachbehandlung in deutlich kürzeren Zeiten zu niedrigeren Restgehalten an freiem Alkylierungsmittel in den kationischen Tensidlösungen führt. Die Erfindung beruht auf der Erkenntnis, daß die Aminosäuren bei erhöhter Temperatur rasch und vollständig mit dem Alkylierungsmittel abreagieren.

### Kationische Tenside

Kationische Tenside im Sinne der Erfindung stellen bekannte chemische Stoffe dar und folgen der allgemeinen Formel **(I)**, 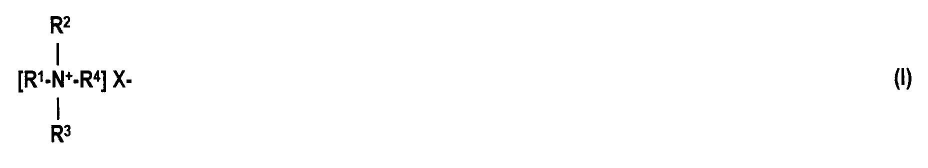 in der R¹, R² und R³ unabhängig voneinander für lineare oder verzweigte, gegebenenfalls auch hydroxysubstituierte Alkylreste mit 1 bis 18 Kohlenstoffatomen oder Alkenylreste mit 8 bis 18 Kohlenstoffatomen, R⁴ für einen linearen oder verzweigten Alkylrest mit 1 bis 4 Kohlenstoffatome oder einen Phenylrest und X für ein Halogenidion, vorzugsweise Chlorid oder Bromid, oder das Anion der Methylschwefelsäure steht.

### Alkylierungsmittel

Unter Alkylierungsmittel sind hierbei Benzylchlorid, Dimethylsulfat und insbesondere Methylchlorid zu verstehen.

### Aminosäuren

Als Aminosäuren mit 2 bis 8 Kohlenstoffatomen, die für die Nachbehandlung der kationischen Tensidlösungen in Betracht kommen, eignen sich Alanin, Arginin, Asparagin, Cystein, Cystin, Dibromtyrosin, Diiodtyrosin, Glutamin, Glutaminsäure, Histidin, Hydroxylysin, Hydroxyprolin, Isoleucin, Leucin, Lysin, Methionin, Phenylalanin, Prolin, Serin, Threonin, Thyroxin, Tryptophan, Tyrosin und Valin. Bevorzugt wird die Nachbehandlung mit Glycin durchgeführt.

### Nachbehandlung

Die Nachbehandlung der wäßrigen Lösungen kationischer Tenside kann durch Zusatz 0,1 bis 10, vorzugsweise 0,5 bis 3 Gew.-%, bezogen auf die Tensidlösung, der Aminosäuren erfolgen. Bevorzugt wird die Nachbehandlung mit 0,5 bis 3,0 Gew.-%, bezogen auf die Tensidlösung, einer 25 bis 80 Gew.%igen wäßrigen und/oder alkoholischen Glycinlösung durchgeführt. Die Nachbehandlung der kationischen Tensidlösungen erfolgt, indem man die Lösungen mit dem Nachbehandlungsmittel über einen Zeitraum von 1 bis 4, vorzugsweise 1 bis 2 h, bei einem pH-Wert von 7,5 bis 9,5, der sich automatisch einstellt, und einer Temperatur von 60 bis 98, vorzugsweise 70 bis 90°C erwärmt. Allgemein gilt, daß die Senkung des Restgehaltes an Alkylierungsmittel um so rascher erfolgt, je höher die Temperatur bei der Nachbehandlung ist. Die Nachbehandlung erfolgt in der Regel bei Normaldruck. Zur Beschleunigung der Reaktion ist jedoch möglich, bei erhöhtem Druck, beispielsweise von 1 bis 2 bar, und einer Temperatur von bis zu 120°C im Druckautoklaven zu arbeiten. Durch die Nachbehandlung wird der Restgehalt an freiem Alkylierungsmittel, insbesondere Methylchlorid innerhalb von 1 bis 4 h auf Werte unterhalb 5 ppm, bezogen auf den Feststoffgehalt der Tensidlösung, herabgesetzt.

### Beispiele

**Ausgangsstoff.** Als Ausgangsstoff wurde eine 25 Gew.-%ige wäßrige Lösung von Cetyltrimethylammoniumchlorid (A), erhältlich durch Alkylierung von Cetyldimethylamin mit Methylchlorid, eingesetzt. Der Gehalt an freiem Methylchlorid betrug 900 ppm.

**Beispiel 1.** In einem 500 ml Dreihalskolben mit Rührer, Rückflußkühler und Innenthermometer wurden 200 g (0,6 mol) A vorgelegt und mit 0,5 g (entsprechend 1 Gew.-% bezogen auf den Feststoffgehalt der kationischen Tensidlösung) Glycin versetzt. Die Reaktionsmischung wurde bei pH = 8 bis 8,5 und einer Temperatur von T = 90°C über einen Zeitraum von t = 1 h erhitzt und anschließend auf Raumtemperatur abgekühlt. Der Restgehalt an Methylchlorid wurde gaschromatographisch ermittelt. Die Ergebnisse sind in Tabelle 1 zusammengefaßt.

**Vergleichsbeispiele V1 bis V3.** In einer Apparatur analog Beispiel 1 wurden 200 g A ohne Glycinzusatz bei pH = 8 bis 8,5, T = 90°C und t = 1 bis 3 h thermisch nachbehandelt und der Restgehalt an Methylchlorid ermittelt. Die Ergebnisse sind in Tabelle 1 zusammengefaßt.

**Tabelle 1**

| **Nachbehandlung von kationischen Tensidlösungen** | | | | |
|---|---|---|---|---|
| **Bsp.** | **Aminosäure** | **Einsatzmenge** **Gew.-%** | **t** **h** | **Methylchlorid-Gehalt** **ppm** |
| 1 | Glycin | 1 | 1 | <5 |
| V1 | - | - | 1 | 140 |
| V2 | - | - | 2 | 43 |
| V3 | - | - | 3 | 23 |

## Patentansprüche

1. Verfahren zur Verminderung des Restgehaltes an freiem Alkylierungsmittel in wäßrigen Lösungen kationischer Tenside, **dadurch gekennzeichnet**, daß man die Lösungen mit einer Aminosäure mit 2 bis 8 Kohlenstoffatomen bei erhöhter Temperatur und gegebenenfalls erhöhtem Druck nachbehandelt.

2. Verfahren nach Anspruch 1, **dadurch gekennzeichnet**, daß man die Lösungen mit Glycin nachbehandelt.

3. Verfahren nach den Ansprüchen 1 und 2, **dadurch gekennzeichnet**, daß man die Lösungen mit 0,1 bis 10 Gew.-% - bezogen auf die Tensidlösung - einer Aminosäure nachbehandelt.

4. Verfahren nach den Ansprüchen 1 bis 3, **dadurch gekennzeichnet**, daß man die Lösungen über einen Zeitraum von 1 bis 4 h, bei einem pH-Wert von 7,5 bis 9,5 und einer Temperatur von 60 bis 98°C nachbehandelt.

## Claims

1. A process for reducing the residual content of free alkylating agent in aqueous solutions of cationic surfactants, characterized in that the solutions are aftertreated at elevated temperature and, optionally, under elevated pressure with an amino acid containing 2 to 8 carbon atoms.

2. A process as claimed in claim 1, characterized in that the solutions are aftertreated with glycine.

3. A process as claimed in claims 1 and 2, characterized in that the solutions are aftertreated with 0.1 to 10% by weight, based on the surfactant solution, of an amino acid.

4. A process as claimed in claims 1 to 3, characterized in that the solutions are aftertreated over a period of 1 to 4 h at a temperature of 60 to 98°C and at a pH value of 7.5 to 9.5.

## Revendications

1. Procédé pour diminuer la teneur résiduelle en agent d'alkylation libre dans des solutions aqueuses d'agents tensioactifs cationiques,
caractérisé en ce que
l'on retraite les solutions avec un aminoacide ayant de 2 à 8 atomes de carbone à température élevée et le cas échéant sous pression accrue.

2. Procédé selon la revendication 1,
caractérisé en ce que
l'on retraite les solutions avec de la glycine.

3. Procédé selon les revendications 1 et 2,
caractérisé en ce que
l'on retraite les solutions avec de 0,1 à 10 % en poids (rapporté à la solution d'agent tensioactif) d'un aminoacide.

4. Procédé selon les revendications 1 à 3,
caractérisé en ce que
l'on retraite les solutions pendant une durée de 1 à 4 heures, à une valeur de pH de 7,5 à 9,5 et à une température de 60 à 98°C.
